# EUROPÄISCHE PATENTSCHRIFT

(11) **EP 1 475 047 B1**
(45) Veröffentlichungstag und Bekanntmachung des Hinweises auf die Patenterteilung: **10.12.2008**
(21) Anmeldenummer: 04007555.8
(22) Anmeldetag: 29.03.2004
(51) Int. Cl.: A61B 17/22

(54) **Stosswellenquelle mit einem piezoelektrischen Ultraschalltransducer**
Shock wave generator with a piezoelectric ultrasonic transducer
Source d'ondes de choc avec un transducteur piezo-électrique à ultrasons

(30) Priorität: 25.04.2003 DE 10318853
(43) Veröffentlichungstag der Anmeldung: 10.11.2004
(73) Patentinhaber: Dornier MedTech Systems GmbH, 82234 Wessling (DE)
(72) Erfinder: Eizenhöfer, Harald, 82229 Seefeld (DE)
(74) Vertreter: Grünecker, Kinkeldey, Stockmair & Schwanhäusser Anwaltssozietät

(56) Entgegenhaltungen:
- DE-A1- 19 653 085
- DE-U1- 8 809 253
- US-A- 5 203 334
- US-A- 5 699 804

## Beschreibung

Die Erfindung betrifft eine Stoßwellenquelle nach dem Oberbegriff von Anspruch 1.

Ein derartiger Ultraschalltransducer ist beispielsweise aus der US 5,203,334 bekannt. Der Ultraschalltransducer ist Bestandteil eines Lithotripters und dient zur Bildgewinnung von einem Bereich, in dem beispielsweise Nieren- oder Gallensteine zerstört werden sollen.

Der Ultraschalltransducer ist in einem Gehäuse angeordnet, das durch Tragarme, die seitlich angeordnet sind, gehalten wird. Durch einen der Tragarme verläuft die elektrische Anschlussleitung zu dem piezoelektronischen Ultraschalltransducer.

Bei dieser Anordnung hat es sich als nachteilig herausgestellt, dass der Tragarm, in dem die Anschlussleitung untergebracht ist, die ungehinderte Ausbreitung von den Stoßwellen, die in der Stoßwellenquelle erzeugt werden, behindert.

Der generelle Aufbau einer Funkenstrecke mit zwei gegenüberliegenden Elektrodenspitzen, welche in einer Fokussierkammer angeordnet sind, wird in der DE 33 16 837 beschrieben. Die DE 196 53 085 zeigt den funktionellen Aufbau einer Ultraschallwandlereinrichtung bzw. eines Ultraschalltransducers, welcher aus einem in dem Transducergehäuse gelagerten ein- oder zweidimensionalen Array von Wandlerelementen besteht, die über eine Platine und flexible elektrische Leitungen, welche im Inneren des Transducergehäuses verlaufen, mit Energie versorgt werden.

Die G 88 09 253 beschreibt einen Stoßwellengenerator zum berührungslosen Zertrümmern von Konkrementen im Körper eines Lebewesens bei welcher ein Schallapplikator zwischen einer Stoßwellenquelle und einer akustischen Sammellinse angeordnet ist. Der Schallapplikator wird dabei mit Hilfe von mehreren Armen in seiner Position gehalten und über separate elektrische Leitungen, welche außerhalb der Arme verlaufen, mit Energie versorgt.

Aufgabe der vorliegenden Erfindung ist es daher, eine Stoßwellenquelle zur Verfügung zu stellen, mit der eine Behinderung der Ausbreitung der Stoßwellen so gut wie möglich verhindert wird.

Diese Aufgabe wird mit einer Stoßwellenquelle nach Anspruch 1 gelöst.

Bei der Stoßwellenquelle ist der elektrische Anschluss für den Ultraschalltransducer, der zur Bildgewinnung dient, so flach ausgebildet und so angeordnet, dass es möglich ist, einen umhüllenden Tragarm vorzusehen, die nur eine minimale Abschattung der Stoßwelle verursacht.

Neben dem Tragarm mit dem Anschlussleiter können auch noch weitere Tragarme für den Ultraschalltransducer vorgesehen sein.

Der flache Anschlussleiter kann beispielsweise ein Flachbandkabel, eine Platine oder eine flexible Platine ("flexboard") sein. Diese eigen sich gut, um durch einen schmalen Tragarm hindurch geführt zu werden. Außerhalb des Bereichs des Tragarms oder auch außerhalb der Stoßwellenquelle geht der elektrische Anschlussleiter vorteilhafterweise in ein konventionelles Anschlusskabel über. Ein solches Kabel erlaubt das Bewegen der Stoßwellenquelle, um diese richtig zu positionieren, und gewährleistet einen Schutz der elektrischen Leitungen vor mechanischen Einwirkungen.

Vorteilhaft ist weiterhin eine Ausführungsform, bei der die äußere Kontur des Tragarmprofils abgerundete Ecken aufweist. Dies vermeidet eine allzu starke Streuung von Stoßwellen an Ecken oder Kanten.

Einige besondere Ausführungsformen des erfindungsgemäßen piezoelektrischen Ultraschalltransducers und der erfindungsgemäßen Stoßwellenquelle werden anhand der beiliegenden Figuren erläutert. Dabei zeigt:
Fig. 1 eine dreidimensionale schematische Darstellung einer Stoßwellenquelle,
Fig. 2 eine schematische Querschnittsansicht des Tragarms.

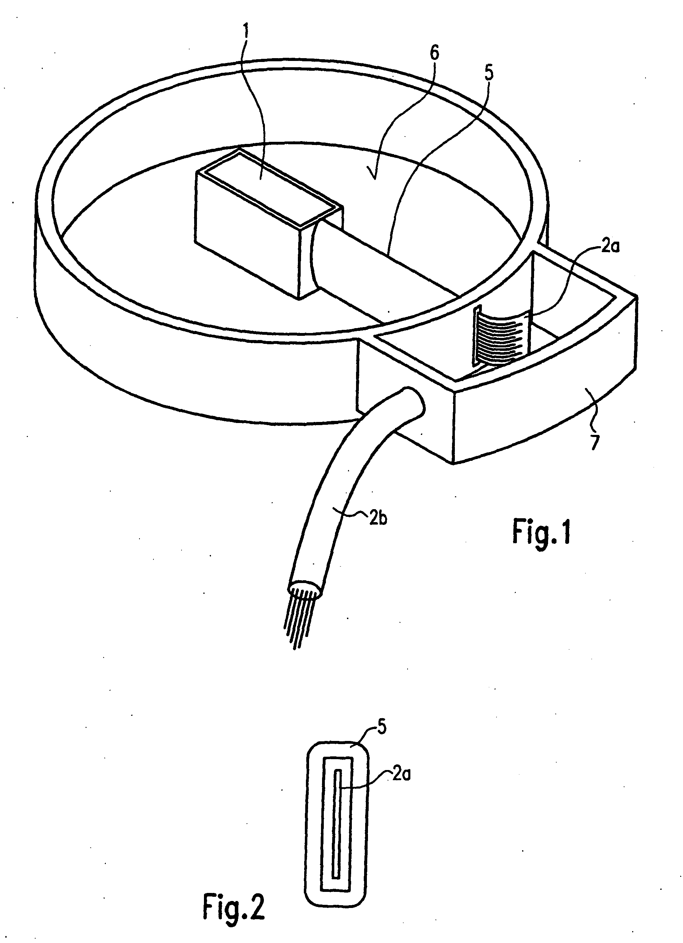

In Fig. 1 ist der schematische Aufbau einer Stoßwellenquelle gezeigt. Die Stoßwellenquelle weist beispielsweise einen flächigen Stoßwellenerzeuger 6 auf. Jedoch sind auch andere Stoßwellenerzeuger möglich. Die Stoßwellenquelle 6 kann Stoßwellen in Fig. 1 nach oben abgeben. Vor dem Stoßwellenerzeuger 6 ist der piezoelektrische Ultraschalltransducer 1 angeordnet, der über einen Tragarm 5 gehalten wird. Der piezoelektrische Ultraschalltransducer 1 kann hierbei ein Gehäuse aufweisen oder in einem Gehäuse angeordnet sein, das den Ultraschalltransducer 1 vor den Stoßwellen und dem Stoßwellenausbreitungsmedium (Fluid) schützt. Mittels des Tragarms 5 kann der Ultraschalltransducer 1 mittig vor dem Stoßwellenerzeuger 6 angeordnet werden.

Wie in Fig. 1 zu sehen, ist der Tragarm 5 wesentlich höher als breit. Ein Querschnitt dieses Tragarms 5 ist in Fig. 2 dargestellt. Innerhalb des Tragarms 5 ist der elektrische Anschlussleiter 2a im Querschnitt dargestellt. Wie in Fig. 2 deutlich zu erkennen, ist der Tragarm 5 hohl, so dass er den elektrischen Leiter 2a aufnehmen kann und darüber hinaus ist der Hohlraum der Form des elektrischen Leiters 2a angepasst. Der Leiter kann, wie in Figur 2 dargestellt, lose in dem Hohlraum angeordnet sein oder auch eingepasst oder an einer oder mehr Seite anliegend oder befestigt.

Der elektrische Anschlussleiter kann ein Flachbandkabel, eine Platine oder eine flexible Platine ("flexboard") sein. Vorteilhaft ist hierbei beispielsweise eine Multilayer-Platine. Sie ist vorteilhafterweise aus einem flexiblen Basismaterial wie Polyimid, oder PTFE (Polytetrafluorethylen) hergestellt.

Die äußere Kontur des Tragarms 5 ist abgerundet, um so die Streuung von Stoßwellen zu minimieren.

Wie in Fig. 1 zu erkennen, ist der flache elektrische Leiter 2a und der flache Tragarm 5 mit seiner großen Ausdehnung im Querschnitt, d. h. mit seiner Höhe parallel zu der Stoßwellenausbreitungsrichtung angeordnet, d. h. senkrecht zu dem flächigen Stoßwellenerzeuger 6.

Weiterhin ist in Fig. 1 zu erkennen, dass der elektrische Leiter 2a aus dem Tragarm 5 nach außen hin herausragt, so dass er mit einem zweiten Leiter 2b verbunden werden kann. Der zweite Leiter 2b ist hierbei ein konventionelles Kabel, mit dem üblicherweise Ultraschalltransducer angeschlossen werden.

Bei der Ausführungsform von Fig. 1 weist die Stoßwellenquelle ein Gehäuse 7 auf, in den der erste Leiter 2a und der zweite Leiter 2b hineinragen, so dass die beiden Leiter dort mit einer Verbindungseinrichtung verbunden werden können. Hierzu können die Adern des zweiten Leiters 2b beispielsweise direkt auf die flexible Platine 2a aufgelötet werden. Auch ist es möglich hier Quetsch- oder Steckkontakte oder ähnliches vorzusehen, um so eine lösbare Verbindung zu erhalten.

Insgesamt ergibt sich durch die flache Ausführung des Leiters 2a, der mit dem Ultraschalltransducer verbunden ist, die Möglichkeit einen ebenfalls flachen Tragarm 5 vorzusehen, der somit nur einen minimalen Querschnitt für die Stoßwellenausbreitung aufweist und somit eine freie Ausbreitung der Stoßwellen nur minimal behindert.

## Patentansprüche

1. Stoßwellenquelle, welche Stoßwellen in einer Stoßwellenrichtung abgibt, mit einem piezoelektrischen Ultraschalltransducer der an einem Tragarm (5) befestigt ist, durch den ein elektrischer Anschlussleiter (2a) zum Verbinden des Ultraschalltransducers mit einem Ansteuergerät geführt ist,
**dadurch gekennzeichnet, dass**
der Tragarm (5) flach ist und mit seiner großen Ausdehnung im Querschnitt im Wesentlichen parallel zu der Stoßwellenausbreitungsrichtung angeordnet ist.

2. Stoßwellenquelle nach Anspruch 1, **dadurch gekennzeichnet, dass** der flache Anschlussleiter (2a) ein Flachbandkabel, eine Platine oder eine flexible Platine umfasst oder ist.

3. Stoßwellenquelle nach Anspruch 2, **dadurch gekennzeichnet, dass** die Platine eine Mulitlayer-Platine ist.

4. Stoßwellenquelle nach Anspruch 2 oder 3, **dadurch gekennzeichnet, dass** das Basismaterial der Platine Polyimid oder PTFE (Polytetraflurethylen) umfasst.

5. Stoßwellenquelle nach einem der Ansprüche 1 bis 4, **dadurch gekennzeichnet, dass** der flache Anschlussleiter (2a) an ein mehradriges Kabel (2b) mit einem bevorzugterweise im Wesentlichen runden Querschnitt angeschlossen ist.

6. Stoßwellenquelle nach Anspruch 5, **dadurch gekennzeichnet, dass** der Anschluss außerhalb des Bereichs des Tragarms oder außerhalb der Stoßwellenquelle liegt.

7. Stoßwellenquelle nach einem der Ansprüche 1 bis 6, **dadurch gekennzeichnet, dass** der Tragarm (5) mindestens zweimal so hoch wie breit ist, bevorzugter Weise mindestens dreimal, viermal oder fünfmal so hoch oder noch höher.

8. Stoßwellenquelle nach einem der Ansprüche 1 bis 7, **dadurch gekennzeichnet, dass** der Tragarm (5) im Querschnitt eine abgerundete Kontur aufweist.

## Claims

1. Shock wave source, which emits shock waves in a shock wave direction, with a piezoelectric ultrasonic transducer which is attached to a carrying arm (5) through which an electric connection lead (2a) is passed to connect the ultrasonic transducer with a control device,
**characterized in that**
the carrying arm (5) is flat and, as seen in a cross-sectional view is arranged with its large dimension essentially in parallel to the direction of shock wave propagation.

2. Shock wave source according to claim 1, **characterized in that** the flat connection lead (2a) comprises or is a flat band cable, a board or a flexible board.

3. Shock wave source according to claim 2, **characterized in that** the board is a multi layer board.

4. Shock wave source according to claim 2 or 3, **characterized in that** the base material of the board comprises polyimide or PTFE (polytetrafluorethylene).

5. Shock wave source according to one of claims 1 to 4, **characterized in that** the flat connection lead (2a) is connected to a multi-lead cable (2b) with a cross-section which is preferably essentially round.

6. Shock wave source according to claim 5, **characterized in that** the connection is outside of the area of the carrying arm or outside of the shock wave source.

7. Shock wave source according to one of the claims 1 to 6, **characterized in that** the carrying arm (5) is at least two times higher than it is wide, in a preferable manner at least three times, four times or five times as high or even higher.

8. Shock wave source according to one of the claims 1 to 7, **characterized in that** the carrying arm (5) exhibits a rounded contour in cross-section.

## Revendications

1. Source d'ondes de choc, lesquelles ondes de choc sont émises dans une direction d'ondes de choc, comprenant un transducteur piézoélectrique à ultrasons fixé sur un bras support (5), à travers lequel un conducteur de connexion électrique (2a) est guidé pour connecter le transducteur à ultrasons à un appareil de commande,
**caractérisée en ce que**
le bras support (5) est plat et est agencé avec sa grande dimension dans une section transversale de manière sensiblement parallèle à la direction de propagation des ondes de choc.

2. Source d'ondes de choc selon la revendication 1, **caractérisée en ce que** le conducteur de connexion plat (2a) comprend ou est un câble plat, une carte ou une carte flexible.

3. Source d'ondes de choc selon la revendication 2, **caractérisé en ce que** la carte est une carte multicouche.

4. Source d'ondes de choc selon la revendication 2 ou 3, **caractérisée en ce que** le matériau de base de la carte comprend du polyimide ou du PTFE (polytétrafluoroéthylène).

5. Source d'ondes de choc selon l'une quelconque des revendications 1 à 4, **caractérisée en ce que** le conducteur de connexion plat (2a) est connecté à un câble multiconducteur (2b) ayant de préférence une section transversale sensiblement ronde.

6. Source d'ondes de choc selon la revendication 5, **caractérisée en ce que** la connexion se trouve hors de la zone du bras support ou hors de la source d'ondes de choc.

7. Source d'ondes de choc selon l'une quelconque des revendications 1 à 6, **caractérisée en ce que** le bras support (5) est au moins deux fois plus haut que large, de préférence au moins trois fois, quatre fois ou cinq fois plus haut ou encore plus haut.

8. Source d'ondes de choc selon l'une quelconque des revendications 1 à 7, **caractérisée en ce que** le bras support (5) présente un contour arrondi dans une section transversale.
